# EUROPÄISCHE PATENTSCHRIFT

(11) **EP 1 214 511 B1**
(45) Veröffentlichungstag und Bekanntmachung des Hinweises auf die Patenterteilung: **10.03.2004**
(21) Anmeldenummer: 00966038.2
(22) Anmeldetag: 15.09.2000
(51) Int. Cl.: F02D 41/14, F02D 41/02

(54) **VERFAHREN ZUR FUNKTIONSÜBERWACHUNG EINES IN EINEM ABGASKANAL EINER VERBRENNUNGSKRAFTMASCHINE ANGEORDNETEN NOx-SENSORS**
METHOD FOR MONITORING THE FUNCTIONING OF A NOx SENSOR ARRANGED IN AN EXHAUST GAS CHANNEL OF AN INTERNAL COMBUSTION ENGINE
PROCEDE POUR SURVEILLER LE FONCTIONNEMENT D'UN DETECTEUR DE NOx PLACE DANS LA CONDUITE DES GAZ D'ECHAPPEMENT D'UN MOTEUR A COMBUSTION INTERNE

(30) Priorität: 22.09.1999 DE 19945374
(43) Veröffentlichungstag der Anmeldung: 19.06.2002
(73) Patentinhaber: Volkswagen Aktiengesellschaft, 38436 Wolfsburg (DE)
(72) Erfinder: HAHN, Hermann, 30175 Hannover (DE); HINZE, Sören, 38106 Braunschweig (DE); LANG, Axel, 38304 Wolfenbüttel (DE)
(74) Vertreter: Pohlmann, Bernd Michael
(86) Internationale Anmeldenummer: PCT/EP2000/009071
(87) Internationale Veröffentlichungsnummer: WO 2001/021951

(56) Entgegenhaltungen:
- EP-A- 0 916 941
- DE-A- 19 823 921
- US-A- 5 426 934
- US-A- 5 797 384

## Beschreibung

Die Erfindung betrifft ein Verfahren zur Funktionsüberwachung eines in einem Abgaskanal einer Verbrennungskraftmaschine angeordneten NO_{X}-Sensors mit den im Oberbegriff des Anspruchs 1 genannten Merkmalen.

Zur Reduzierung einer Schadstoffemission von Verbrennungskraftmaschinen ist es bekannt, geeignete Katalysatoren in dem Abgaskanal anzuordnen. Einerseits können in den Katalysatoren Schadstoffe, die als Reduktionsmittel wirken können, wie CO, HC oder H₂, mit Luftsauerstoff oxidiert werden, und andererseits wird ebenfalls während eines Verbrennungsvorganges in der Verbrennungskraftmaschine gebildetes NO_{X} mit Hilfe der Reduktionsmittel an den Katalysatoren zu Stickstoff reduziert.

Befindet sich die Verbrennungskraftmaschine in einem verbrauchsgünstigeren Magerbetrieb, so ist der Sauerstoffanteil am Luft-Kraftstoff-Gemisch erhöht, und infolgedessen ist ein Anteil der Reduktionsmittel am Abgas verringert. Damit kann allerdings auch nicht mehr eine ausreichende Umsetzung von NOₓ gewährt werden. Zur Abhilfe ist in dem Abgaskanal ein NO_{X}-Speicher angeordnet, der mit dem Katalysator zu einem NO_{X}-Speicherkatalysator zusammengefaßt werden kann. Der NO_{X}-Speicherkatalysator absorbiert NO_{X} so lange, bis entweder eine NO_{X}-Desorptionstemperatur überschritten oder eine NO_{X}-Speicherfähigkeit erschöpft ist. Vor diesem Zeitpunkt muß demnach ein Wechsel in einen Regenerationsbetrieb mit λ ≤ 1 zur Regeneration des NO_{X}-Speicherkatalysators stattfinden, um eine NO_{X}-Emission zu verhindern.

Es ist bekannt, eine Regenerationsnotwendigkeit von einer stromab des NO_{X}-Speicherkatalysators erfaßten NO_{X}-Konzentration abhängig zu machen. Die NO_{X}-Konzentration wird mittels eines NO_{X}-Sensors erfaßt. Nachteilig ist hierbei jedoch, daß beim Vorliegen einer Fehlfunktion des NO_{X}-Sensors zu hohe NO_{X}-Emissionen

Aus der US 5426934 A ist ein Verfahren zur Funktionsüberwachung eines in einem Abgaskanal einer Verbrennungskraftmaschine angeordneten Sensors bekannt, wobei der Sensor stromab eines Dreiwegkatalysators angeordnet ist und das Funktionsprüfungsverfahren des NOx-Sensors die folgenden Schritte aufweist:
- Feststellung, ob der Motor mit einem im wesentlichen stöchiometrischen Luft-/Kraftstoffverhältnis (Lambda-Stöch) arbeitet
- Lesung und Speicherung des stöchiometrischen Wertes (N_{stöch}) vom NOx-Sensor
- Feststellung, ob der Motor im wesentlichen mit einem vorgegebenen mageren Luft-/Kraftstoffverhältnis (Lambda_{mager}) arbeitet
- Lesung anschließend und Speicherung des mageren Wertes (N_{mager}) vom NOx-Sensor
- Berechnung eines Signalverhältnisses SR gemäß der Formel SR = N_{mager}/N_{stöch}
- Vergleichung des berechneten Wertes von SR mit einem im voraus festgelegten Schwellenwert für SR und
- Erzeugung eines Anzeige-Ausgangssignals, wenn der berechnete Wert von SR kleiner als der Schwellenwert von SR ist.

Der Erfindung liegt die Aufgabe zugrunde, derartige Fehlfunktionen des NO_{X}-Sensors in einfacher Weise zu erfassen, um dann gegebenenfalls geeignete Gegenmaßnahmen ergreifen zu können.

Erfindungsgemäß wird diese Aufgabe durch das Verfahren zur Funktionsüberwachung des NO_{X}-Sensors mit den in den Ansprüchen 1 und 5 genannten Merkmalen gelöst. Dadurch, daß
(a) innerhalb eines Diagnosezeitraums anhand eines Messsignals des NOx-Sensors (18) und einer NOx-Rohemission der Verbrennungskraftmaschine (10) eine vom NOx-Speicherkatalysator (16) absorbierte NOx-Masse ermittelt wird,
(b) gleichzeitig anhand eines Modells für den NO_{X}-Speicherkatalysator eine absorbierte NO_{X}-Sollmasse berechnet wird und
(c) ein Verhältnis der NO_{X}-Masse zur NO_{X}-Sollmasse (Kontrollwert KWₙ) mit einem unteren Grenzwert Gₙᵤ oder einem oberen Grenzwert Gₙₒ verglichen wird,
oder dadurch, daß
(a) eine Dauer tₘₑₛ für eine vollständige NO_{X}-Regeneration des NO_{X}-Speicherkatatysators erfaßt wird,
(b) anhand eines Modells für den NO_{X}-Speicherkatalysator und einem gemessenen oder berechneten NO_{X}-Beladungszustand eine Solldauer t_{mod} für die NO_{X}-Regeneration berechnet wird und
(c) ein Verhältnis der Dauer tₘₑₛ zur Solldauer t_{mod} (Kontrollwert KWₜ) mit einem unteren Grenzwert G_{tw} oder einem oberen Grenzwert Gₜₒ verglichen wird,
kann in einfacher Weise die Funktionsüberwachung des NO_{X}-Sensors erfolgen.

In einer bevorzugten Ausgestaltung des Verfahrens werden beim Überschreiten des Kontrollwertes KWₙ beziehungsweise KWₜ über die oberen Grenzwerte Gₙₒ, Gₜₒ oder beim Unterschreiten der unteren Grenzwerte Gₙᵤ, Gₜᵤ Wartungssignale erzeugt. Nach dem Auftreten eines solchen Wartungssignales kann dann durch geeignete Wartungsmaßnahmen der Fehler behoben werden, oder es wird gegebenenfalls der NO_{X}-Sensor ausgetauscht.

Weiterhin ist vorteilhaft, den Diagnosezeitraum derart festzulegen, daß er unmittelbar nach einer vollständigen NO_{X}-Regeneration des NO_{X}-Speicherkatalysators und einem Wechsel in den Magerbetrieb der Verbrennungskraftmaschine beginnt. Vorteilhafterweise endet der Diagnosezeitraum nach einer Feststellung der Regenerationsnotwendigkeit des NO_{X}-Speicherkatalysators oder mit einem Wechsel in den Regenerationsbetrieb.

Die Funktionsüberwachung des NO_{X}-Sensors sollte in bevorzugter Weise nur dann stattfinden, wenn ein weitestgehend konstant verlaufender Magerbetrieb in der Verbrennungskraftmaschine detektiert wurde. Auf diese Weise können die schwer zu berücksichtigenden Einflüsse eines dynamischen Betriebes der Verbrennungskraftmaschine auf das Speicherkatalysatormodell vermieden werden.

Weitere bevorzugte Ausgestaltungen der Erfindung ergeben sich aus den übrigen, in den Unteransprüchen genannten Merkmalen.

Die Erfindung wird nachfolgend in einem Ausführungsbeispiel anhand der zugehörigen Zeichnungen näher erläutert. Es zeigen:
- Figur 1: eine schematische Anordnung einer Verbrennungskraftmaschine mit einem NO_{X}-Speicherkatalysator und einem NO_{X}-Sensor und
- Figur 2: ein Blockschaltbild für eine Funktionsüberwachung des NO_{X}-Sensors gemäß dem Ausführungsbeispiel.

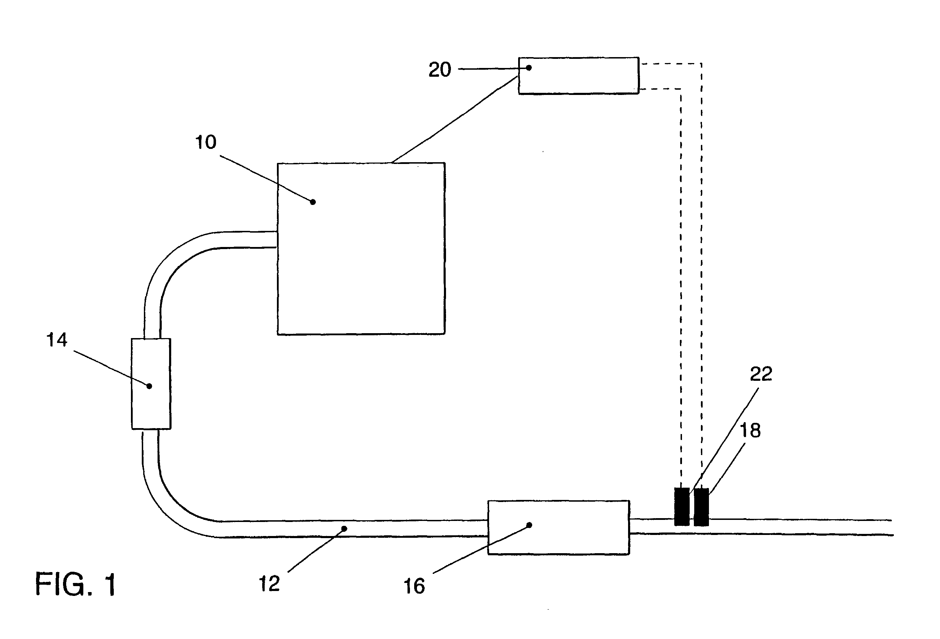

Die Figur 1 zeigt eine Anordnung einer Verbrennungskraftmaschine 10, die in einem Abgaskanal 12 einen Vorkatalysator 14 und einen NO_{X}-Speicherkatalysator 16 aufweist. Der Vorkatalysator 14 und der NO_{X}-Speicherkatalysator 16 dienen zur Minderung einer Schadstoffemission der Verbrennungskraftmaschine 10.

Üblicherweise weisen dazu die Katalysatoren 14, 16 Katalysatorkomponenten auf, die eine Oxidation von gebildeten Reduktionsmitteln, wie CO, HC oder H₂, mit Luftsauerstoff ermöglichen. Zumindest der NO_{X}-Speicherkatalysator 16 weist eine Katalysatorkomponente auf, die eine Reduktion von ebenfalls während eines Verbrennungsvorganges eines Luft-Kraftstoff-Gemisches gebildetem NO_{X} mittels der Reduktionsmittel ermöglicht. Befindet sich die Verbrennungskraftmaschine 10 allerdings in einem Magerbetrieb, so reicht in der Regel ein Anteil der Reduktionsmittel am Abgas nicht aus, um eine hinreichend hohe Umsetzung von NO_{X} zu gewähren. Im Magerbetrieb wird daher durch eine Speicherkomponente des NO_{X}-Speicherkatalysators 16 das NO_{X} als Nitrat absorbiert.

Die Absorption des NO_{X} kann nur so lange erfolgen, bis entweder eine NO_{X}-Desorptionstemperatur überschritten wird oder eine NO_{X}-Speicherfähigkeit erschöpft ist. Vor diesem Zeitpunkt muß demnach ein Wechsel in einen Regenerationsbetrieb mit λ ≤ 1 stattfinden, um eine NO_{X}-Regeneration zu ermöglichen. Ausschlaggebend für eine Regenerationsnotwendigkeit kann dabei in bekannter Weise eine durch den NO_{X}-Sensor 18 erfaßte NO_{X}-Konzentration beziehungsweise NO_{X}-Emission sein. Ein entsprechendes Meßsignal wird dazu beispielsweise an ein Motorsteuergerät 20 weitergeleitet, dort ausgewertet und zur Steuerung eines Arbeitsmodus der Verbrennungskraftmaschine 10 verwendet.

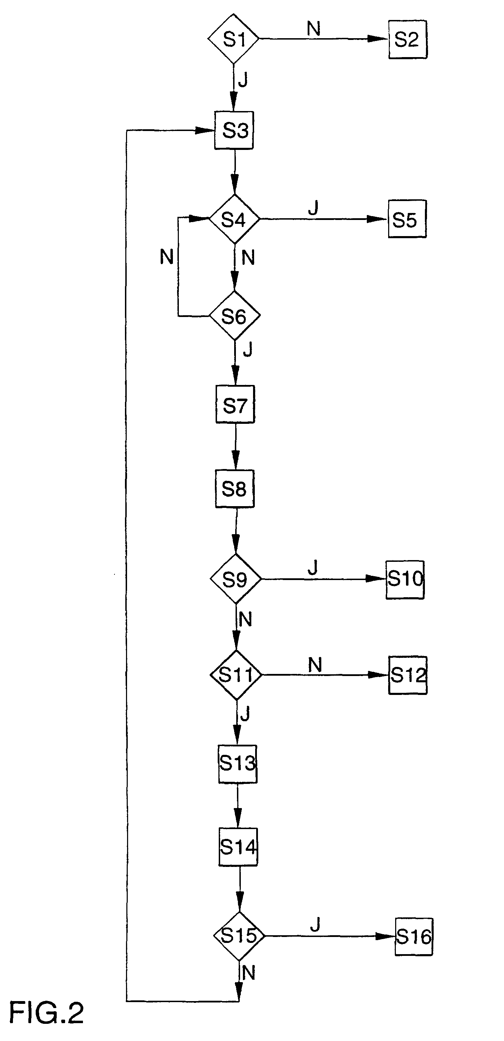

Die Figur 2 zeigt ein Blockschaltbild, mit dem eine Funktionsüberwachung des NO_{X}-Sensors 18 während eines dynamischen Betriebes der Verbrennungskraftmaschine 10 gemäß diesem Ausführungsbeispiel erfolgen kann. In einem Schritt S1 wird zunächst erfaßt, ob eine vollständige NO_{X}-Regeneration des NO_{X}-Speicherkatalysators 16 durchgeführt wurde. Ist dies nicht der Fall, so wird die Funktionsüberwachung des Sensors 18 abgebrochen (Schritt S2).

Mit Beginn des Magerbetriebs (Schritt S3) startet gleichzeitig eine Ermittlung einer in den NO_{X}-Speicherkatalysator 16 eingelagerten NO_{X}-Masse. Dazu wird zum einen während eines vorgegebenen Diagnosezeitraums mittels des NO_{X}-Sensors 18 die NO_{X}-Konzentration stromab des NO_{X}-Speicherkatalysators 16 erfaßt und aufsummiert und anschließend von einer gemessenen oder berechneten NO_{X}-Rohemission der Verbrennungskraftmaschine 10 abgezogen. Zum anderen wird mit Hilfe bekannter Modelle des NO_{X}-Speicherkatalysators 16 und anhand der NO_{X}-Rohemission eine absorbierte NO_{X}-Sollmasse berechnet. Die NO_{X}-Sollmasse entspricht maximal einer NO_{X}-Masse, die von einem frischen NO_{X}-Speicherkatalysator 16 absorbiert werden kann.

In einem Schritt S4 wird kontinuierlich überprüft, ob sich die Verbrennungskraftmaschine 10 während des Diagnosezeitraums in einem konstant verlaufenden Magerbetrieb befindet. Bei Störungen infolge dynamischer Vorgänge, beispielsweise durch ein Wechseln in einen Homogenbetrieb oder eine Schubabschaltung, ist die für den Diagnosezeitraum berechnete NO_{X}-Sollmasse besonders fehlerbehaftet, und es erfolgt daher ein Abbruch der Funktionsüberwachung (Schritt S5). Vorzugsweise wird der Diagnosezeitraum derart festgelegt, daß er - wie bereits erläutert - mit dem Wechsel in den Magerbetrieb (Schritt S3) beginnt und so lange fortgeführt wird, bis eine Regenerationsnotwendigkeit detektiert wird (Schritt S6).

Eine derartige Regenerationsnotwendigkeit kann beispielsweise über den NO_{X}-Sensor 18 in Form einer Schwellenemission für NO_{X} detektiert werden. Nachdem die Regenerationsnotwendigkeit vorliegt, wird ein Wechsel in den Regenerationsbetrieb mit λ ≤ 1 initiiert (Schritt S7). Gleichzeitig wird ein Zeitzähler gestartet, mit dem eine Dauer tₘₑₛ für eine vollständige NO_{X}-Regeneration ermittelt werden soll.

Aus einem Verhältnis der über den NO_{X}-Sensor 18 für den NO_{X}-Speicherkatalysator 16 ermittelten absorbierten NO_{X}-Masse und der NO_{X}-Sollmasse wird in einem Schritt S8 ein Kontrollwert KWₙ gebildet. Überschreitet der Kontrollwert KWₙ in einem Schritt S9 einen oberen Grenzwert Gₙₒ oder unterschreitet einen unteren Grenzwert Gₙᵤ, so liegt ein Defekt des NO_{X}-Sensors 18 vor und ein Wartungssignal wird erzeugt (Schritt S10). Der obere Grenzwert GWₒ wird üblicherweise derart gewählt, daß er ein Verhältnis der über den NO_{X}-Sensor 18 ermittelten NO_{X}-Masse zu der NO_{X}-Sollmasse in einem frischen NO_{X}-Speicherkatalysator 16 wiedergibt.

Ist der Kontrollwert KWₙ zwischen den beiden Grenzwerten Gₙᵤ, Gₙₒ, so kann in einem Schritt S11 geprüft werden, ob die NOₓ-Regeneration vollständig durchgeführt wurde. Dazu eignet sich beispielsweise eine Lambdasonde 22, die stromab des NO_{X}-Speicherkatalysators 16 angeordnet ist. Gegen Ende der NO_{X}-Regeneration sinkt der Lambdawert deutlich ab, und es kann beispielsweise durch Vorgabe eines geeigneten Schwellenwertes ein Stopsignal für den Zeitzähler gesetzt werden (Schritt S13). Wird die NO_{X}-Regeneration vorzeitig unterbrochen, erfolgt auch hier ein Abbruch der Funktionsüberwachung des NO_{X}-Sensors 18 (Schritt S12).

Mit Hilfe des Speicherkatalysatormodells wird aus einem gemessenen oder berechneten NO_{X}-Beladungszustand eine Solldauer t_{mod} für die NO_{X}-Regeneration berechnet. Ein Verhältnis der Dauer tₘₑₛ zur Solldauer t_{mod} liefert einen Kontrollwert KWₜ (Schritt S14). Der Kontrollwert KWₜ wird mit einem oberen Grenzwert Gₜₒ oder einem unteren Grenzwert Gₜᵤ in einem Schritt S15 verglichen. Überschreitet der Kontrollwert KWₜ den oberen Grenzwert Gₜₒ oder unterschreitet den unteren Grenzwert Gₜᵤ, so liegt ein Sensordefekt vor, und es wird ein Wartungssignal erzeugt (Schritt S16). Ist dies nicht der Fall, so kann ein neuer Zyklus der Funktionsüberwachung, beginnend mit dem Schritt S3, eingeleitet werden. Der obere Grenzwert Gₜₒ wird wiederum derart gewählt, daß er ein Verhältnis der Dauer tₘₑₛ zur Solldauer t_{mod} in einem frischen NO_{X}-Speicherkatalysator 16 wiedergibt.

Auch wird die Sensor-Plausibilität dahingehend geprüft, ob sich zum Beispiel mit schlechterem Einspeicherverhalten des Katalysators nicht nur eine geringere gemessene Füllung ergibt, sondern sich gleichzeitig auch in entsprechendem Maße die benötigte gemessene Regenerationszeit verringert.

## Patentansprüche

1. Verfahren zur Funktionsüberwachung eines in einem Abgaskanal einer Verbrennungskraftmaschine angeordneten NOx-Sensors, der stromab eines NOx-Speicherkatalysators angeordnet ist, **dadurch gekennzeichnet, dass**
(a) innerhalb eines Diagnosezeitraums anhand eines Messsignals des NOx-Sensors (18) und einer NOx-Rohemission der Verbrennungskraftmaschine (10) eine vom NOx-Speicherkatalysator (16) absorbierte NOx-Masse ermittelt wird,
(b) gleichzeitig anhand eines Modells für den NOx-Speicherkatalysator (16) eine absorbierte NOx-Sollmasse berechnet wird und
(c) ein Verhältnis der NOx-Masse zur NOx-Sollmasse (Kontrollwert KWₙ) mit einem unteren Grenzwert (Gₙᵤ) oder einem oberen Grenzwert (Gₙₒ) verglichen wird.

2. Verfahren nach Anspruch 1, **dadurch gekennzeichnet, dass** beim Überschreiten des Kontrollwerts (KWₙ) über den oberen Grenzwert (Gₙₒ) oder beim Unterschreiten des unteren Grenzwertes (Gₙᵤ) ein Wartungssignal erzeugt wird.

3. Verfahren nach den Ansprüchen 1 oder 2, **dadurch gekennzeichnet, dass** der Diagnosezeitraum unmittelbar nach einer vollständigen NOx-Regeneration des NOx-Speicherkatalysators (16) und einem Wechsel in einen Magerbetrieb der Verbrennungsmaschine (10) beginnt.

4. Verfahren nach Anspruch 3, **dadurch gekennzeichnet, dass** der Diagnosezeitraum nach einer Feststellung einer Regenerationsnotwendigkeit des NOx-Speicherkatalysators (16) oder mit einem Wechsel in einen Regenerationsbetrieb endet.

5. Verfahren zur Funktionsüberwachung eines in einem Abgaskanal einer Verbrennungskraftmaschine angeordneten NOx-Sensors, der stromab eines NOx-Speicherkatalysators angeordnet ist, **dadurch gekennzeichnet, dass**
(a) eine Dauer (tₘₑₛ) für eine vollständige NOx-Regeneration des NOx-Speicherkatalysators (16) erfasst wird,
(b) anhand eines Modells für den NOx-Speicherkatalysator (16) und einem gemessenen oder berechneten NOx-Beladungszustand eine Solldauer (t_{mod}) für die NOx-Regeneration berechnet wird und
(c) ein Verhältnis der Dauer (tₘₑₛ) zur Solldauer (t_{mod}) (Kontrollwert KWₜ) mit einem unteren (Gₜᵤ) oder einem oberen Grenzwert (Gₜₒ) verglichen wird.

6. Verfahren nach Anspruch 5, **dadurch gekennzeichnet, dass** beim Überschreiten des Kontrollwerts (KWₜ) über den oberen Grenzwert (Gₜₒ) oder beim Unterschreiten des unteren Grenzwerts (Gₜᵤ) ein Wartungssignal erzeugt wird.

7. Verfahren nach einem der vorhergehenden Ansprüche, **dadurch gekennzeichnet, dass** eine Funktionsüberwachung des NOx-Sensors (19) lediglich nach einem weitestgehend konstant verlaufenden Magerbetrieb erfolgt.

## Claims

1. Method for monitoring the functioning of an NOx sensor which is arranged in an exhaust gas channel of an internal combustion engine, downstream of an NOx storage catalytic converter, **characterized in that**
(a) an NOx mass absorbed by the NOx storage catalytic converter (16) is determined within a diagnosis period on the basis of a measurement signal from the NOx sensor (18) and of untreated NOx emission from the internal combustion engine (10),
(b) at the same time, a desired absorbed NOx mass is calculated on the basis of a model for the NOx storage catalytic converter (16), and
(c) a ratio of the NOx mass to the desired NOx mass (control value KWₙ) is compared with a lower limit value (Gₙᵤ) or an upper limit value (Gₙₒ).

2. Method according to Claim 1, **characterized in that** a maintenance signal is generated in the event of the control value (KWₙ) exceeding the upper limit value (Gₙₒ) or dropping below the lower limit value (Gₙᵤ).

3. Method according to Claim 1 or 2, **characterized in that** the diagnosis period begins immediately after complete NOx regeneration of the NOx storage catalytic converter (16) and a changeover to a lean-burn mode of the internal combustion engine (10).

4. Method according to Claim 3, **characterized in that** the diagnosis period ends after it has been established that the NOx storage catalytic converter (16) needs to be regenerated or in the event of a changeover to a regeneration mode.

5. Method for monitoring the functioning of an NOx sensor which is arranged in an exhaust gas channel of an internal combustion engine, downstream of an NOx storage catalytic converter, **characterized in that**
(a) a duration (tₘₑₛ) for complete NOx regeneration of the NOx storage catalytic converter (16) is recorded,
(b) a desired duration (t_{mod}) for the NOx regeneration is calculated on the basis of a model for the NOx storage catalytic converter (16) and a measured or calculated NOx loading state, and
(c) a ratio of the duration (tₘₑₛ) to the desired duration (t_{mod}) (control value KWₜ) is compared with a lower limit value (Gₜᵤ) or an upper limit value (Gₜₒ).

6. Method according to Claim 5, **characterized in that** a maintenance signal is generated in the event of the control value (KWₜ) exceeding the upper limit value (Gₜₒ) or dropping below the lower limit value (Gₜᵤ).

7. Method according to one of the preceding claims, **characterized in that** monitoring of the functioning of the NOx sensor (19) only takes place after an as far as possible constant lean-burn mode.

## Revendications

1. Procédé destiné à surveiller le fonctionnement d'un capteur de NOₓ, disposé dans le conduit d'évacuation des gaz d'échappement d'un moteur à combustion interne, lequel est disposé en aval d'un catalyseur accumulateur de NOₓ, **caractérisé en ce que**
(a) une masse de NOₓ absorbée par le catalyseur accumulateur de NOₓ (16) est détectée au cours d'une période de diagnostic à l'aide d'un signal de mesure du capteur de NOₓ (18) et d'une émission de NOₓ brut par le moteur à combustion interne,
(b) simultanément, une masse de consigne absorbée pour le NOₓ est calculée à l'aide d'un modèle pour le catalyseur accumulateur de NOₓ (16) et
(c) un rapport entre la masse de NOₓ et la masse de consigne pour le NOₓ (valeur de contrôle KWₙ) est comparé avec une valeur limite inférieure (Gₙᵤ) ou une valeur limite supérieure (Gₙₒ).

2. Procédé selon la revendication 1, **caractérisé en ce qu'**un signal de maintenance est généré lors du dépassement de la valeur de contrôle (KWₙ), au-delà de la valeur limite supérieure (Gₙₒ) ou lorsque la valeur limite inférieure (Gₙᵤ) n'est pas atteinte.

3. Procédé selon l'une quelconque des revendications 1 ou 2, **caractérisé en ce que** la période de diagnostic commence directement après une régénération complète du NOₓ dans le catalyseur accumulateur de NOₓ (16) et après un passage du moteur à combustion interne (10) en régime pauvre.

4. Procédé selon la revendication 3, **caractérisé en ce que** la période de diagnostic s'achève après la détection d'une nécessité de régénération du catalyseur accumulateur de NOₓ (16) ou après un passage en mode de régénération.

5. Procédé destiné à surveiller le fonctionnement d'un capteur de NOₓ, disposé dans le conduit d'évacuation des gaz d'échappement d'un moteur à combustion interne, lequel est disposé en aval d'un catalyseur accumulateur de NOₓ, **caractérisé en ce que**
(a) une durée (tₘₑₛ) correspondant à une régénération complète du NOₓ sur le catalyseur accumulateur de NOₓ (16) est détectée,
(b) une durée de consigne (t_{mod}) pour la régénération du NOₓ est calculée à l'aide d'un modèle pour le catalyseur accumulateur de NOₓ (16) et d'un état de charge de NOₓ mesuré ou calculé et
(c) un rapport entre la durée (tₘₑₛ) et la durée de consigne (t_{mod}) (valeur de contrôle KWₜ) est comparé avec une valeur limite inférieure (Gₜᵤ) ou une valeur limite supérieure (Gₜₒ).

6. Procédé selon la revendication 5, **caractérisé en ce qu'**un signal de maintenance est généré lors du dépassement de la valeur de contrôle (KWₜ), au-delà de la valeur limite supérieure (Gₜₒ) ou lorsque la valeur limite inférieure (Gₜᵤ) n'est pas atteinte.

7. Procédé selon l'une quelconque des revendications précédentes, **caractérisé en ce qu'**une surveillance du fonctionnement du capteur de NOₓ (19) n'est réalisée qu'après un régime pauvre à déroulement largement constant.
